# EUROPEAN PATENT SPECIFICATION

(11) **EP 3 930 640 B1**
(45) Date of publication and mention of the grant of the patent: **09.08.2023**
(21) Application number: 20714687.9
(22) Date of filing: 28.02.2020
(51) Int. Cl.: A61F 5/02, A61F 5/30

(54) **ORTHOPAEDIC BRACE AND METHOD FOR CUSTOMISING AN ORTHOPAEDIC BRACE**
ORTHOPÄDISCHE STÜTZE UND VERFAHREN ZUR ANPASSUNG EINER ORTHOPÄDISCHEN STÜTZE
ATTELLE ORTHOPÉDIQUE ET PROCÉDÉ DE PERSONNALISATION D'UNE ATTELLE ORTHOPÉDIQUE

(30) Priority: 01.03.2019 IT 201900003045
(43) Date of publication of application: 05.01.2022
(73) Proprietor: Isico S.r.l., 20141 Milano (IT); Tessadri, Fabrizio, 38052 Caldonazzo (TN) (IT)
(72) Inventor: NEGRINI, Stefano, 20141 Milano (IT); TESSADRI, Fabrizio, 38052 Caldonazzo (TN) (IT)
(74) Representative: Bernotti, Andrea
(86) International application number: PCT/IB2020/051717
(87) International publication number: WO 2020/178688

(56) References cited:
- WO-A1-95/19154
- US-A- 4 202 327
- US-A- 4 559 933
- US-A1- 2015 119 780

## Description

### CROSS-REFERENCE TO RELATED APPLICATIONS

This patent application claims priority from Italian patent application no. 102019000003045 filed on 1/03/2019.

### TECHNICAL FIELD

This invention relates to an orthopaedic brace and a method for customising an orthopaedic brace.

### BACKGROUND ART

As is well known, the treatment of certain medium and severe spinal column diseases, including vertebral deformities and in particular scoliosis, involves the use of orthopaedic braces. The orthopaedic braces are shaped to exert forces on the patient's pelvis in order to modify the curvature of the column and the loads weighing on it. Compared to torsos in cast, the mechanical properties of which can be replicated, orthopaedic braces are generally better tolerated, both because the risk of causing sores is lower and because orthopaedic braces have the advantage that they can be put on and taken off relatively easily, for example to enable daily hygiene activities and the changing of underwear. Tolerability for patients is a determining factor for the effectiveness of treatments and may limit the spread of certain types of orthopaedic braces especially.

A limitation of known corsets, especially those with greater stiffness, which are more effective even for the most severe diseases, is linked to the fact that production can only be partially industrialised. The braces currently available, in fact, have almost no possibility of adjusting the respective parts, especially with regard to the socalled pelvis in relation to the torso. The shape of the pelvis is of particular importance in an orthopaedic brace because it must, on the one hand, contribute to the overall stiffness of the structure and, on the other hand, discharge the forces applied to the torso onto the pelvis, without being irritating or painful or tending to cause contact sores over time. Since there is no possibility of adjusting, in particular for the pelvic section, the orthopaedic braces have to be tailor-made for each patient and it is not possible in practice to use industrial techniques that reduce manufacturing costs. Similarly, it is not possible to adapt the shape of the orthopaedic brace when the patient's posture changes as a result of treatment or growth. In practice, it may happen that, due to the patient's changed physical condition, the orthopaedic brace is no longer suitable for properly transferring the forces and, after an initial stage, the treatment loses its optimal effectiveness. This also happens very quickly, particularly at the first application of the brace, since the skeletal structure of the pelvis cannot change, compared to the enormous volumetric variation that can occur at the level of the "waist". It can mean that in the first, delicate stage of treatment, the brace loses effectiveness very quickly.

Therefore, the unchangeable volume of the pelvis, which also increases over time due to the patient's growth, becomes a huge obstacle to the suitable constant corrective increase that the treatment requires and that could be achieved through a progressive closure of the orthosis to match the torso's changed volumes. In this sense, it should be borne in mind that growth implies variations in diameters that are normally not consistent between the pelvis and the torso: the reduced reciprocal adaptability, therefore, requires more frequent brace changes and consequent costs.

In addition, given the complexity of the system of forces in play, even an orthopaedic brace that is initially well designed and made may, over time, give rise to pelvic sores that may discourage the patient from continuing treatment and require adaptation.

Another potentially critical aspect depends on whether the pelvis is to be made from a plaster model or a three-dimensional scan of the patient's body. The procedure requires very high precision and may easily lead to errors if it is not carried out by staff with suitable and particularly advanced training.

US 4 202 307 A is considered to represent the closest prior art, it describes an orthopaedic brace comprising two rigid valves coupled to one another and each having a respective pelvic section and a respective supporting shell. The pelvic sections are rigidly coupled to the supporting shells of the respective valves. Other examples of known orthopaedic braces are described in US 4 559 933 A.

### DISCLOSURE OF INVENTION

The purpose of this invention is to provide an orthopaedic brace and method for customising an orthopaedic brace that makes it possible to overcome, or at least to mitigate, the limitations described.

According to this invention, an orthopaedic brace is provided, therefore, that comprises two rigid valves hinge-coupled to one another and each having a respective pelvic section and a respective supporting shell, wherein the pelvic sections are rigidly coupled to the supporting shells of the respective valves and are at least in part made of a thermoformable polymeric first material having a softening point between 45° C and 80° C.

The low softening point of the thermoformable material makes it possible to make the pelvic sections mouldable at temperatures that the patient can easily tolerate. The forming of the pelvic sections can, therefore, be carried out directly on the patient, obtaining maximum adaptability. In addition, there is no need to make the orthopaedic brace using the exact measurements of the patient from the outset. On the one hand, therefore, this invention favours the industrialisation of the manufacturing process with a consequent reduction in costs and increased accessibility of the tool. In fact, the supporting shells, less critical, may be mass-produced with a limited number of standard moulds that adapt to the needs of a wide range of patients without the need for strong customisation. The pelvic sections, which require greater precision, may be thermoformed directly on the patient. These, too, can therefore be mass produced and then optimally customised by thermoforming. On the other hand, the thermoforming process may be repeated whenever necessary, in particular to adapt the pelvic sections as the patient's shape changes as a result of the treatment, disease and/or growth. In this way, maximum effectiveness can be maintained throughout the whole treatment.

According to one aspect of the invention, the first material has a Shore A hardness greater than 80 and a Shore D hardness greater than 30 at temperatures below the softening point.

The specified hardness guarantees the thermoformable polymeric material's resistance to deformation at operating temperatures, which only exceptionally approach 40 °C. In use, the thermoformable polymeric material gives the necessary stiffness for the correct transferral of forces between the pelvic sections, the support shells, and the patient's body.

According to one aspect of the invention, the thermoformable polymeric first material contains ethylene-vinyl acetate copolymer.

The material defined in this way lends itself particularly well to making customisable pelvic sections because it combines the low softening point and the necessary stiffness below the softening point.

According to one aspect of the invention, the supporting shells are made of a second material having a stiffness greater than the stiffness of the first material.

For the supporting shells, which do not require much customisation, it is a priority to maintain high stiffness to ensure the correct transfer of forces to the patient.

According to one aspect of the invention, the second material is selected so that the supporting shells have a stiffness that is equal to or greater than the stiffness of a body that has the same shape and are made from a plate of high-density polyethylene (HDPE) that is 4 mm thick.

According to one aspect of the invention, the second material has an elastic modulus equal to or greater than 800 MPa and/or a Shore D hardness equal to or greater than 62.

In this way, the properties of the material ensure the effectiveness of the brace supporting shells.

According to one aspect of the invention, the second material is a polymeric material having a softening point greater than the softening point of the first material forming the pelvic sections.

The softening point that is higher than the softening point of the material forming the pelvic sections prevents the torso structure from being compromised by temperature jumps, for example during the thermoforming step of pelvic sections.

According to one aspect of the invention, the pelvic sections comprise respective iliac supports, shaped for contact with a patient's iliac crests, and trochanteric regions shaped for contact with the patient's trochanters.

According to one aspect of the invention, the pelvic sections are connected to the respective supporting shells along junction arcs contouring the iliac supports.

According to one aspect of the invention, the supporting shells comprise respective sacrococcygeal appendixes that extend downward and rearward along the junction arcs in relation to the iliac supports.

According to one aspect of the invention, the supporting shells comprise respective iliopubic appendixes that extend downward and frontward along the junction arcs in relation to the iliac supports.

The sacrococcygeal appendixes and the iliopubic appendixes contribute, individually and in combination, to the stability of the orthopaedic brace and to the effectiveness of the coupling with the patient's pelvis.

According to one aspect of the invention, at least the iliac supports and the trochanteric regions are made of the first material.

To maximise the overall stiffness of the brace, the low temperature thermoformable sections made of the first material may be limited to contact with the regions wherein it is most important that the brace be very precisely shaped to the patient.

According to one aspect of the invention, the supporting shells of the valves are shaped so as to wrap rearward, on one side, and frontward a respective half-torso.

The type of orthopaedic brace, with high stiffness and shaped to act basically on the entire torso of the patient, is particularly effective especially, but not only, for the treatment of cases of medium and severe vertebral deformities and scoliosis.

According to one aspect of the invention, each supporting shell comprises a plurality of sections, made as separate parts and rigidly joined together to form the respective supporting shell.

The production of the supporting shells in several rigidly connected parts makes it possible to customise the supporting shells too without compromising neither the overall stiffness nor the possibility to industrially producing the orthopaedic brace. With a limited and sustainable number of moulds, in fact, it is possible to make each part in different sizes, which may then be selected according to the patient's needs.

According to one aspect of the invention, each supporting shell comprises adjusting members configured to adjust relative positions of the respective portions.

The ability to adjust the distance between the portions of each supporting shell increases the freedom to customise the orthopaedic brace after the manufacturing thereof and, thus, favours its industrial production.

According to another aspect of the invention, there is provided a method for customising an orthopaedic brace, comprising two rigid valves hinge-coupled to one another and each having a respective pelvic section and a respective supporting shell, wherein the pelvic sections are rigidly coupled to the supporting shells of the respective valves and are made of a thermoformable polymeric material having a softening point ranging between 45° C and 80° C, the method comprising:
applying the orthopaedic brace to a patient;
heating the pelvic sections to above the softening point of the thermoformable polymeric material; and
forming the pelvic sections on the patient.

### BRIEF DESCRIPTION OF THE DRAWINGS

Further characteristics and advantages of this invention will become clear from the following description of the non-limiting embodiments thereof, with reference to the accompanying drawings, in which:
- Figure 1 is a rear perspective view of an orthopaedic brace in accordance with one embodiment of this invention;
- Figure 2 is a rear orthogonal view of the orthopaedic brace in Figure 1;
- Figure 3 is a front perspective view of the orthopaedic brace in Figure 1;
- Figure 4 is a front orthogonal view of an orthopaedic brace in accordance with a different embodiment of this invention;
- Figure 5 is a front orthogonal view of an orthopaedic brace in accordance with another embodiment of this invention;
- Figure 6 is a front orthogonal view of an orthopaedic brace in accordance with another embodiment of this invention.

### BEST MODE FOR CARRYING OUT THE INVENTION

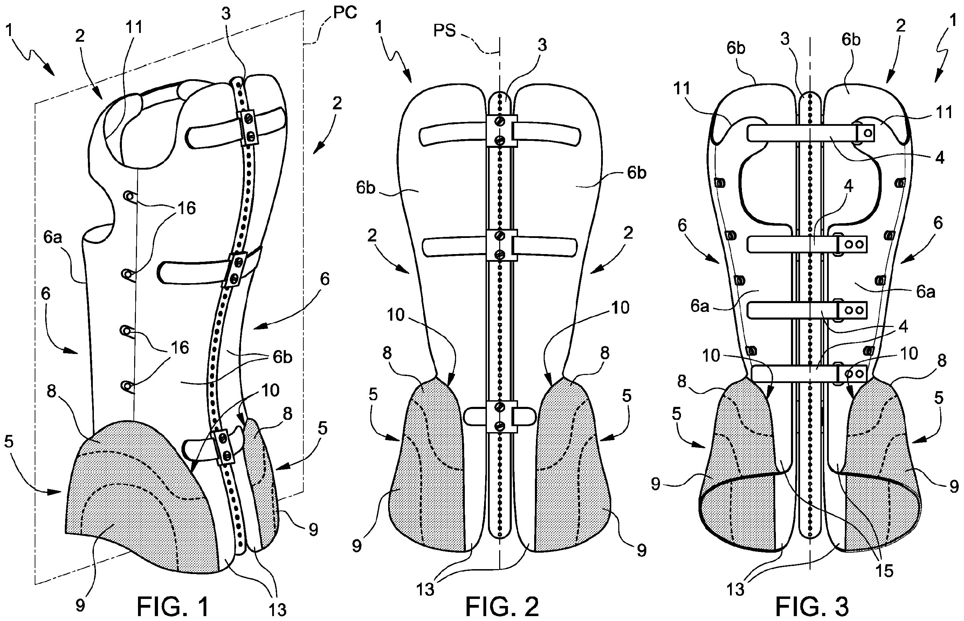

With reference to Figures 1-3, an orthopaedic brace in accordance with an embodiment of this invention is indicated with the reference number 1 and comprises two rigid valves 2 hinge-coupled by means of a spine bar 3, with a hinge axis parallel to a median sagittal plane PS. The term "valve" is derived from the field of zoology, where it indicates each of the two parts forming the shell of some molluscs, and is commonly used in the sector to indicate each of the parts that, in a brace, wrap around and support the patient's torso. In the case of this invention, each valve 2 corresponds to a respective supporting half or main part of the brace 1. It should be understood that the two supporting halves or main parts are not necessarily identical and, on the contrary, may have some structural differences to correct asymmetries typically related to spinal column diseases.

The hinged coupling enables, on the one hand, the opening of the two valves 2 so that the patient can put on and take off the brace 1, and, on the other hand, the maintenance of the stiffness between the two valves 2 without mutual displacements once the brace 1 is closed. More precisely, in the example of Figures 1-3, the spine bar 3 extends on the median sagittal plane PS and is arranged between the valves 2, which are hinged thereto in the respective dorsal regions. The valves 2 are, therefore, opposite each other in relation to the median sagittal plane PS. The hinge coupling, as well as using properly socalled hinges, with pins rotating in seats, may be achieved using belt and buckle systems fixed to one of the valves 2 and to the spine bar 3. In addition, the hinge coupling, in one embodiment, may be made directly between the valves 2, without the spine bar 3. The orthopaedic brace 1 is also equipped with closure members 4, which enable the valves 2 to be tightened at the front in an adjustable way once the patient has put on the orthopaedic brace 1. In one embodiment, the closure members 4 may comprise belts and buckles.

Each valve 2 has a respective pelvic section 5 and a respective supporting shell 6, rigidly coupled to each other. According to the treatment indications, the valves 2 may or may not be symmetrical.

The pelvic sections 5 are caps each surrounding a respective half pelvis of the patient when the orthopaedic brace 1 is put on. The pelvic sections 5 together form a pelvis of the orthopaedic brace 1 and comprise respective iliac supports 8 and trochanteric regions 9. The iliac supports 8 are shaped for contact with a patient's iliac crests, so that the forces exerted by the supporting shells 6 of the orthopaedic brace 1 on the patient's torso along its vertical axis are discharged onto the pelvis.

The pelvic sections 5 are connected to the respective supporting shells 6 along the junction arcs 10 surrounding the iliac supports 8 and extending around the hips. The connection may be made, for example, by welding, gluing, riveting or any technique that is suitable for rigid fastening. The trochanteric regions 9 are shaped for contact with the patient's trochanters, so as to give stability to the brace 1 when worn.

The pelvic sections 5 are made of a first material, which is a thermoformable polymeric material with a softening point (Vicat Softening Point) ranging between 45° C and 80° C. In addition, at temperatures below the softening point (and in particular at temperatures of use that normally do not exceed 35-40° C), the thermoformable polymeric first material with which the pelvic sections 5 are made have a Shore A hardness greater than 80 and a Shore D hardness greater than 30. At operating temperatures, the parameters indicated are suitable for giving the pelvis of the orthopaedic brace 1 enough stiffness to transfer the forces necessary to reduce spinal dysmorphism to the patient's torso.

In one embodiment, the first material contains ethylene-vinyl acetate copolymer and may have the following composition by weight:
Ethylene-vinyl acetate copolymer > 98%;
Processing aids < 2%;
Vinyl acetate CAS number 108-05-4 < 0.3%.

In the embodiment in Figures 1-3, the pelvic sections 5 are made entirely of the thermoformable polymeric first material having the characteristics described above. Alternatively, however, the pelvic sections 5 may comprise parts made of the first material, e.g. the iliac supports 8 and/or the trochanteric regions 9, and parts made of more rigid material, such as the material forming the supporting shells 6.

The supporting shells 6 of the valves 2 are shaped so that they each wrap a respective half (right or left) of the patient's torso at the back, on one side, and at the front, according to individual needs, and extend from the respective pelvic sections 5 as far as necessary along the patient's torso according to medical instructions. In the embodiment represented in Figures 1-3, the supporting shells 6 define axillary supports 11.

In addition, the supporting shells 6 comprise respective sacrococcygeal appendixes 13 and iliopubic appendixes 15, which cooperate with the pelvic sections 5 to give stability and stiffness to the orthopaedic brace 1 when worn. In particular, the sacrococcygeal appendixes 13 extend downwards and rearward along the junction arcs 10 in relation to the iliac supports 8 to cover the patient's sacral region. The iliopubic appendixes 15 extend downward and frontward along the junction arcs 10 in relation to the iliac supports 8.

In one embodiment, moreover, each supporting shell 6 comprises a respective front section 6a and a respective back section 6b, made as separate parts and joined together along a coronal plane PC. The relative position of the front section 6a and of the back section 6b of each valve 6 in the direction perpendicular to the coronal plane PC may be adjusted by adjusting members 16. The adjusting members 16 in one embodiment are adjusting screws, but could, alternatively, comprise tensioners, hooks and racks, belt and buckle systems and any other suitable devices.

In an embodiment not illustrated, however, the supporting shells 6 may each be made of a single piece.

The supporting shells 6 are made of a second material with greater stiffness than the first material forming the pelvic sections 5. In addition, the second material is selected so that the supporting shells 6 maintain their stiffness basically unchanged even during the thermoforming steps of the pelvic sections 5, as described below. In general, the second material may be advantageously selected so that the supporting shells 6 have a stiffness equal to or greater than the stiffness of a body that has the same shape and is made from a plate of high-density polyethylene (HDPE) that is 4 mm thick. In addition, the second material has an elastic modulus equal to or greater than 800 MPa and/or a Shore D hardness equal to or greater than 62.

In one embodiment, the second material may be a polymeric material with the features indicated and having a softening point greater than the softening point of the first material forming the pelvic sections 5. For example, the second material has a softening point equal to or greater than 100° C, preferably equal to or greater than 130° C. In one embodiment, in particular, the supporting shells 6 of the valves 2 are made of high-density polyethylene (HDPE). In a different embodiment, the supporting shells of the valves 2 may be made of a composite material comprising carbon fibres embedded in a matrix.

The orthopaedic brace 1 may be made with a small number of standard moulds and then customised by thermoforming the pelvis, which requires high precision in order to correctly discharge the forces onto the patient's pelvis.

After the orthopaedic brace 1 has been placed on the patient, the pelvic sections 5 are heated and brought above the softening point of the thermoformable material. Heating may be achieved by using jets of hot air. Alternatively, the pelvic sections may be pre-heated in the oven before the orthopaedic brace 1 is placed on the patient. A temperature of a few degrees above the softening point may be enough for the purpose. In the example described, the pelvic sections 5 can be brought to a temperature ranging between 50°C and 60°C, which enables the pelvic sections 5 to be shaped directly on the patient and are normally well tolerated. A layer of fabric, e.g. cotton, may be placed between the patient and the orthopaedic brace 1 before heating to reduce the risk of discomfort without compromising the precision of the forming operations.

The procedure may be carried out when the brace 1 is put on for the first time, until maximum correction is achieved, and may then be repeated whenever it is advisable, for example some time after the start of treatment, when the patient changes his or her posture due to recovery from the dysmorphism.

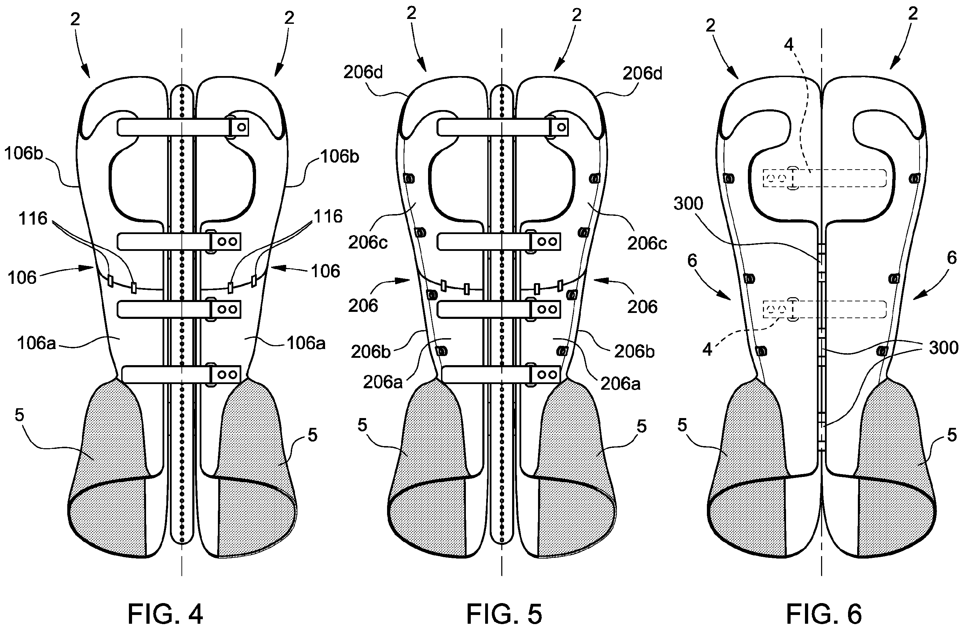

According to one embodiment of the invention, illustrated in Figure 4, the valves 2 comprise respective pelvic sections 5, as already described, and respective supporting shells, here indicated with the number 106. Each supporting shell 106 comprises a respective lumbar section 106a and a respective thoracic section 106b, rigidly coupled to each other. The connection may be permanent, e.g. by bonding or welding, or temporary, e.g. by screw connections 116, which may also serve as adjustment means to adjust the relative position of the lumbar section 106a and the thoracic section 106b along the patient's vertical axis. The pelvic sections 5 are rigidly connected to the lumbar sections 106a of the respective supporting shells 106.

According to one embodiment, illustrated in Figure 5, the valves 2 comprise respective pelvic sections 5, as already described, and respective supporting shells 206. Each supporting shell 206 comprises a respective lumbar section and a respective thoracic section, each of which in turn includes a front section and a back section. In practice, each supporting shell 206 comprises one front lumbar section 206a, one back lumbar section 206b, one front thoracic section 206c and one back thoracic section 206c, all connected to form a single rigid body.

With reference to Figure 6, in another embodiment of the invention, the valves 2, each comprising a pelvic section 5 and a supporting shell 6, are hinged together at the front by hinges 300 and are closed by closure members 4 arranged at the back in the dorsal region.

It is clear, finally, that modifications and variations may be made to the orthopaedic brace described herein while remaining within the scope of protection defined by the attached claims.

In particular, in all embodiments described, any combinations of the materials indicated for the pelvic sections and for the supporting shells may be used. In addition, in any case, the pelvic sections can be entirely made of the thermoformable polymeric first material, or only in certain regions, in particular the iliac supports and/or the trochanteric regions.

## Claims

1. An orthopaedic brace comprising two rigid valves (2) coupled to one another and each having a respective pelvic section (5) and a respective supporting shell (6), wherein the pelvic sections (5) are rigidly coupled to the supporting shells (6) of the respective valves (2), **characterised in that** the two rigid valves are hinge-coupled to one another, and the pelvic sections (5) are at least in part made of a thermoformable polymeric first material having a softening point between 45° C and 80° C.

2. The orthopaedic brace according to claim 1, wherein the first material has Shore A hardness greater than 80 and Shore D hardness greater than 30 at temperatures lower than the softening point.

3. The orthopaedic brace according to claim 1 or 2, wherein the first material contains ethylene-vinyl acetate copolymer.

4. The orthopaedic brace according to any one of the preceding claims, wherein the supporting shells (6) are made of a second material having a stiffness greater than the stiffness of the first material.

5. The orthopaedic brace according to claim 4, the second material is selected so that the supporting shells (6) have stiffness equal to or greater than a stiffness of a body having equal shape and made from a plate of high-density polyethylene (HDPE) with thickness of 4 mm.

6. The orthopaedic brace according to claim 4 or 5, wherein the second material has elastic modulus equal to or greater than 800 MPa and/or Shore D hardness equal to or greater than 62.

7. The orthopaedic brace according to any one of claims 4 to 6, wherein the second material is a polymeric material having a softening point greater than the softening point of the first material forming the pelvic sections (5).

8. The orthopaedic brace according to any one of the preceding claims, wherein the pelvic sections (5) comprise respective iliac supports (8), shaped for contact with iliac crests of a patient, and trochanteric regions (9) shaped for contact with trochanters of the patient.

9. The orthopaedic brace according to claim 8, wherein the pelvic sections are connected to the respective supporting shells (6) along junction arcs (10) contouring the iliac supports (8).

10. The orthopaedic brace according to claim 9, wherein the supporting shells (6) comprise respective sacrococcygeal appendixes (13), that extend downward and rearward along the junction arcs (10) with respect to the iliac supports (8) and respective iliopubic appendixes (15), that extend downward and frontward along the junction arcs (10) with respect to the iliac supports (8).

11. The orthopaedic brace according to any one of claims 8 to 10, wherein at least the iliac supports (8) and the trochanteric regions (9) are made of the first material.

12. The orthopaedic brace according to any one of the preceding claims, wherein the supporting shells (6) of the valves (2) are shaped so as to wrap rearward, on one side and frontward a respective half-torso.

13. The orthopaedic brace according to any one of the preceding claims, wherein each supporting shell (6; 106; 206) comprises a plurality of respective portions (6a, 6b; 106a, 106b; 206a, 206b, 206c, 206d), provided as separate parts and rigidly joined to one another to form the respective supporting shell (6; 106; 206).

14. The orthopaedic brace according to claim 11, wherein each supporting shell (6; 106; 206) comprises adjusting members (16; 116) configured to adjust relative positions of the respective portions (6a, 6b; 106a, 106b; 206a, 206b, 206c, 206d).

15. The orthopaedic brace according to any one of the preceding claims, comprising a rigid spine bar (3) extending in a median sagittal plane (PS) between the valves (2) and wherein the valves (2) are hinged to the spine bar (3) in respective dorsal regions.

16. A method for customizing an orthopaedic brace according to claim 1, the method comprising:
applying the orthopaedic brace to a patient;
heating the pelvic sections (5) to above the softening point of the thermoformable polymeric material; and
forming the pelvic sections (5) onto the patient.

## Patentansprüche

1. Orthopädische Stütze, umfassend zwei miteinander gekoppelte starre Klappen (2), jede aufweisend einen respektiven Beckenabschnitt (5) und eine respektive Stützhülle (6), wobei die Beckenabschnitte (5) starr mit den Stützhüllen (6) der respektiven Klappen (2) gekoppelt sind,
**dadurch gekennzeichnet, dass**
die beiden starren Klappen miteinander scharniergekoppelt sind, und die Beckenabschnitte (5) zumindest zum Teil gefertigt sind aus einem thermoformbaren polymeren ersten Material, aufweisend einen Erweichungspunkt zwischen 45° C und 80° C.

2. Orthopädische Stütze nach Anspruch 1, wobei das erste Material eine Shore-A-Härte von größer als 80 und eine Shore-D-Härte von größer als 30 bei einer unter dem Erweichungspunkt liegenden Temperatur aufweist.

3. Orthopädische Stütze nach Anspruch 1 oder 2, wobei das erste Material Ethylen-Vinylacetat-Copolymer umfasst.

4. Orthopädische Stütze nach einem der vorhergehenden Ansprüche, wobei die Stützhüllen (6) aus einem zweiten Material gefertigt sind, aufweisend eine Steifigkeit, welcher größer ist, als die Steifigkeit des ersten Materials.

5. Orthopädische Stütze nach Anspruch 4, wobei das zweite Material so ausgewählt ist, dass die Stützhüllen (6) eine Steifigkeit aufweisen, welche gleichgroß oder größer ist, als die Steifigkeit eines Körpers, aufweisend dieselbe Gestalt, und gefertigt aus einer Platte High-Density Polyethylene (HDPE) mit einer Dicke von 4 mm.

6. Orthopädische Stütze nach Anspruch 4 oder 5, wobei das zweite Material einen Elastizitätsmodul von gleich oder größer als 800 Mpa und/oder Shore-D-Härte von gleich oder größer als 62 aufweist.

7. Orthopädische Stütze nach einem der Ansprüche 4 bis 6, wobei das zweite Material ein polymeres Material ist, aufweisend einen Erweichungspunkt welcher größer ist, als der des die Beckenabschnitte (5) bildenden ersten Materials.

8. Orthopädische Stütze nach einem der vorhergehenden Ansprüche, wobei die Beckenabschnitte (5) respektive Beckenstützen (8), geformt für den Kontakt mit dem Beckenkamm eines Patienten, und Trochanter-Bereiche (9), geformt für den Kontakt mit Trochantern des Patienten, umfassen.

9. Orthopädische Stütze nach Anspruch 8, wobei die Beckenabschnitte mit den respektiven Stützhüllen (6) entlang von die Beckenstützen (8) konturierenden Verbindungsbögen (10) verbunden sind.

10. Orthopädische Stütze nach Anspruch 9, wobei die Stützhüllen (6) respektive Sakrokozygealanhänge (13), welche sich in Bezug auf die Beckenstützen (8) entlang der Verbindungsbögen (10) nach unten und nach hinten erstrecken, und respektive iliopubische Anhänge (15), welche sich in Bezug auf die Beckenstützen (8) entlang der Verbindungsbögen (10) nach unten und nach vorne erstrecken, umfassen.

11. Orthopädische Stütze nach einem der Ansprüche 8 bis 10, wobei zumindest die Beckenstützen (8) und die Trochanter-Bereiche (9) aus dem ersten Material gefertigt sind.

12. Orthopädische Stütze nach einem der vorhergehenden Ansprüche, wobei die Stützhüllen (6) der Klappen (2) so geformt sind, dass sie auf einer Seite nach hinten und auf der anderen Seite nach vorne jeweils einen halben Torso umschließen.

13. Orthopädische Stütze nach einem der vorhergehenden Ansprüche, wobei jede der Stützhüllen (6, 106, 206) eine Vielzahl respektiver Abschnitte (6a, 6b, 106a, 106b, 206a, 206b, 206c, 206d) umfasst, bereitgestellt als separate Teile und fest miteinander verbunden, um die respektiven Stützhülle (6, 106, 206) auszubilden.

14. Orthopädische Stütze nach Anspruch 11, wobei jede Stützhülle (6, 106, 206) Einstellmittel (16, 116) umfasst, dazu eingerichtet Relativpositionen der respektiven Abschnitte (6a, 6b, 106a, 106b, 206a, 206b, 206c, 206d) einzustellen.

15. Orthopädische Stütze nach einem der vorhergehenden Ansprüche, umfassend eine starre Wirbelsäulenstange (3), welche sich in einer mittleren Sagittalebene (PS) zwischen den Klappen (2) erstreckt und wobei die Klappen (2) in jeweiligen dorsalen Bereichen gelenkig an die Wirbelsäulenstange (3) angeschlossen sind.

16. Verfahren zum Anpassen einer orthopädischen Stütze nach Anspruch 1, umfassend:
Anlegen der orthopädischen Stütze an einen Patienten,
Erwärmen der Beckenabschnitte (5) auf über den Erweichungspunkt des thermoformbaren polymeren Materials; und
Formen der Beckenabschnitte (5) auf den Patienten.

## Revendications

1. Attelle orthopédique comprenant deux valves rigides (2), couplées l'une à l'autre et ayant chacune une section pelvienne (5) respective, ainsi qu'une coque de support (6), respective,
les sections pelviennes (5) étant couplées rigidement aux coques de support (6) des valves (2) respectives,
attelle **caractérisée en ce que**
les deux valves rigides sont couplées par des charnières l'une à l'autre, et les sections pelviennes (5) sont au moins en partie réalisées en une première matière polymère thermoformable, ayant un point de ramollissement compris entre 45°C et 80°C.

2. Attelle orthopédique selon la revendication 1,
dans laquelle
la première matière a une dureté shore A supérieure à 80 et une dureté shore D supérieure à 30 à des températures inférieures au point de ramollissement.

3. Attelle orthopédique selon la revendication 1 ou 2,
dans laquelle
la première matière contient un polymère d'acétate éthylène-vinyl.

4. Attelle orthopédique selon l'une des revendications précédentes,
dans laquelle
les coques de support (6) sont réalisées en une seconde matière ayant une dureté supérieure à la dureté de la première matière.

5. Attelle orthopédique selon la revendication 4,
dans laquelle
la seconde matière est choisie de façon que les coques de support (6) présentent une dureté égale ou supérieure à la dureté d'un corps ayant une même forme et réalisé dans une plaque de polymère haute densité (HDPE) d'une épaisseur de 4 mm.

6. Attelle orthopédique selon la revendication 4 ou 5,
dans laquelle
la seconde matière a un module d'élasticité égal ou supérieur à 800 MPa et/ou une dureté shore D égale ou supérieure à 62.

7. Attelle orthopédique selon l'une des revendications 4 à 6,
dans laquelle
la seconde matière est un polymère ayant un point de ramollissement supérieur au point de ramollissement de la première matière, formant les sections pelviennes (5).

8. Attelle orthopédique selon l'une des revendications précédentes,
dans laquelle
les sections pelviennes (5) ont des supports iliaques (8) formés pour être en contact avec les crêtes iliaques et les régions trochantériennes (9) formées pour être en contact avec les trochanters du patient.

9. Attelle orthopédique selon la revendication 8,
dans laquelle
les régions pelviennes sont reliées aux coques de support (6) respectives, selon des arcs de jonction (10) contournant les supports iliaques (8).

10. Attelle orthopédique selon la revendication 9,
dans laquelle
les coques de support (6) ont des appendices sacro-coccygiens (13) qui dirigés vers le bas et vers l'arrière le long des arcs de jonction (10) par rapport aux supports iliaques (8) et aux appendices iliopubiques (15) respectifs qui s'étendent vers le bas et vers l'avant, selon les arcs de jonction (10) par rapport aux supports iliaques (8).

11. Attelle orthopédique selon l'une des revendications 8 à 10,
dans laquelle
au moins les supports iliaques (8) et les régions trochantériennes (9) sont réalisés dans la première matière.

12. Attelle orthopédique selon l'une des revendications précédentes,
dans laquelle
les coques de support (6) des valves (2) sont formées pour envelopper la partie arrière sur un côté et vers l'avant d'un demi-torse respectif.

13. Attelle orthopédique selon l'une des revendications précédentes, dans laquelle
chaque coque de support (6 ; 106 ; 206) comprend un ensemble de parties respectives (6a, 6b ; 106a, 106b ; 206a, 206b, 206c, 206d) comme pièces distinctes et réunies rigidement l'une à l'autre pour former la coque de support (6 ; 106 ; 206) respective.

14. Attelle orthopédique selon la revendication 11,
dans laquelle
chaque coque de support (6 ; 106 ; 206) a des éléments de réglage (16 ; 116) configurés pour régler les positions relatives des parties respectives (6a, 6b ; 106a, 106b ; 206a, 206b, 206c, 206d).

15. Attelle orthopédique selon l'une des revendications précédentes, comprenant
une barre vertébrale rigide (3) s'étendant dans le plan sagittal médian (PS) entre les valves (2), et
les valves (2) sont articulées à la barre vertébrale (3) dans les régions dorsales respectives.

16. Procédé d'adaptation d'une attelle orthopédique selon la revendication 1,
procédé consistant à :
- appliquer l'attelle orthopédique sur le patient,
- chauffer les sections pelviennes (5) au-dessus du point de ramollissement du polymère thermoformable, et
- former les sections pelviennes (5) sur le patient.
